# EUROPEAN PATENT SPECIFICATION

(11) **EP 1 222 906 B1**
(45) Date of publication and mention of the grant of the patent: **17.10.2007**
(21) Application number: 01200119.4
(22) Date of filing: 15.01.2001
(51) Int. Cl.: A61F 13/15

(54) **Absorbent product such as a sanitary towel, an incontinence pad, a panty liner or the like**
Absorbierendes Produkt wie Binde, Inkontinenzschutz Slipeinlage oder dergleichen
Produit absorbant tel qu'une serviette hygiénique, une protection contre l'incontinence, une garniture de slip ou autre article similaire

(43) Date of publication of application: 17.07.2002
(73) Proprietor: SCA Hygiene Products AB, 405 03 Göteborg (SE)
(72) Inventor: Franzén, Eva, 426 76 Västra Flölunda (SE); Michon-Gente, Marie-Josée, 414 52 Göteborg (SE); Peurasaari, Helena, 435 37 Mölnlycke (SE); Utas Hansson, Marie, 437 31 Lindome (SE); Burchhardt, Tina, 3450 Alleroed (DK)
(74) Representative: Olsson, Stefan

(56) References cited:
- WO-A-00/59426
- WO-A-00/72790
- WO-A-98/57608
- DE-A- 19 837 090
- DE-A- 19 903 109
- DE-U- 20 002 192
- DE-U- 29 904 464
- DE-U- 29 915 071
- DE-U- 29 917 726

## Description

### TECHNICAL FIELD

The present invention relates to an absorbent product, such as a sanitary towel, an incontinence pad, a panty liner or the like, which product has a generally elongate shape with a longitudinal direction and a transverse direction, an upper side and a lower side. The absorbent product also has a first end portion intended to be directed forwards on the wearer and a second end portion intended to be directed backwards on the wearer.

### BACKGROUND ART

Conventional absorbent products of this type usually have a rectangular or hourglass-shaped appearance, and they are usually fastened in a pair of briefs before use by means of, for example, self-adhesive glue. During use of the product, it is held in contact against the body by means of the pressure from the briefs. The lower side of the product is often designed with a liquid-impermeable backing layer in order to prevent liquid penetrating through the product and down into the briefs.

One problem with this type of absorbent product is that it hinders air permeation during use, because the product, when it is fastened in a pair of briefs, can be said to form a barrier which prevents air circulation between the genital area of the wearer and the briefs. Poor air permeation can result in the product becoming warm, moist and uncomfortable to wear, with an increased risk of fungal infection, unpleasant odour or skin irritation as a consequence.

A proposed solution to the above-mentioned problem is that the absorbent products are designed with a breathable backing layer which consists of, for example, microporous plastic films or impervious non-woven material. Examples of breathable materials are given in EP 0 813 848, EP 0 813 849, EP 0 710 472, EP 0 025 315 and US 4,713,069. The disadvantage of this type of breathable backing layer is that it does not have a satisfactory liquid-blocking effect. It may also be mentioned that even products with breathable backing layers can feel warm, moist and uncomfortable during use.

### BRIEF DESCRIPTION OF THE INVENTION

The object of the present invention is to remedy the above-mentioned problems and to construct an absorbent product which reduces the risk of genital discomfort and at the same time feels fresh and safe for the wearer.

An absorbent product according to the present invention is set out in claim 1. The product has a limited extent in the longitudinal direction and the transverse direction and a geometry which is such that the product does not cover as large a part of the genital area as conventional products.

According to the invention, the length of the product lies within the range 65-100 mm and the maximum width of the product in the transverse direction lies within the range 35-55 mm. Furthermore, the width in the transverse direction of the second end portion is to be smaller than the width in the transverse direction of the first end portion. The width of the product in the transverse direction at a point along a longitudinal centre line extending in the longitudinal direction which lies at a distance of 15% of the length of the product from an end line which runs in the transverse direction of the product and is tangent to the second end portion is to be 60-75% of the width of the product in the transverse direction at a point along the longitudinal centre line which lies at a distance of 80% of the length of the product from the end line.

### DESCRIPTION OF THE INVENTION

By means of the present invention, an absorbent product of the type referred to in the introduction has been produced, which product essentially eliminates the difficulties referred to above.

Conventional absorbent products of this type normally have an extent which by and large covers the whole genital area of the wearer, which results in it being difficult for a large part of the moisture which is generated to be ventilated through the product even though the product has a breathable backing layer. The product then feels moist and uncomfortable to wear because it forms a barrier which prevents air permeation between the wearer and the briefs. A study of conventional shaped panty liners available on the market shows that they have a length which lies within the range 140-186 mm and a maximum width in the transverse direction which lies within the range 54-83 mm.

By instead limiting the extent of the product in the longitudinal direction and the transverse direction and by designing the product with a suitable geometry so that a smaller part of the genital area of the wearer is covered by the product, the positive effects of better air permeation being obtained are achieved, which affords a fresher sensation and a reduced risk of problems in the genital area at the same time as the product continues to be safe without a greater risk of wetting-through.

It has been found that a length of the product within the range 65-100 mm and a maximum width in the transverse direction of 35-55 mm is sufficient to obtain good liquid-absorbing capacity and safe protection. It is also important to design the product so that it does not feel unwieldy or uncomfortable to wear, for which reason it should have a geometry which fits the briefs in which it is to be worn and also ensures that the product shapes itself in a suitable manner according to the genital area of the wearer. A suitable design of the product is for the width in the transverse direction of the second end portion to be smaller than the width in the transverse direction of the first end portion and for the width of the product in the transverse direction at a point along a longitudinal centre line extending in the longitudinal direction which lies at a distance of 15% of the length of the product from an end line which runs in the transverse direction of the product and is tangent to the second end portion to be 60-75% of the width of the product in the transverse direction at a point along the longitudinal centre line which lies at a distance of 80% of the length of the product from the end line.

### BRIEF DESCRIPTION OF THE FIGURES

The invention is described in greater detail below with reference to the exemplary embodiments which are shown in the drawings, in which
Figure 1 shows a panty liner according to the invention seen from above;
Figures 2a and 2b show sections along the line II-II through the panty liner in Figure 1;
Figure 3 shows a embossed panty liner according to the invention seen from above,and
Figure 4 shows a panty liner according to an embodiment seen from below.

### DESCRIPTION OF FIGURES AND EMBODIMENTS

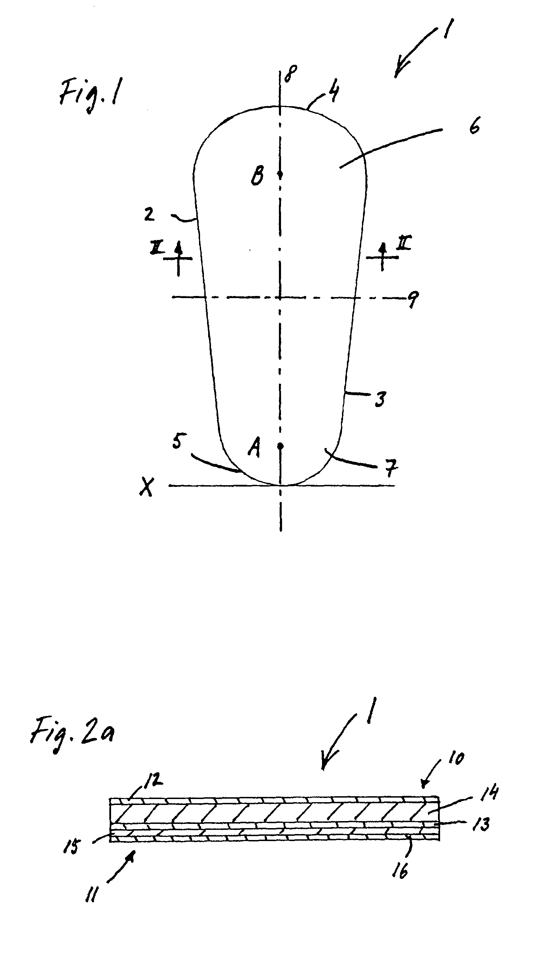

The panty liner 1 in Figures 1 and 2 has a generally elongate shape with a longitudinal direction and a transverse direction and has two long sides 2, 3, two short sides 4, 5, a first end portion 6 intended to be directed forwards on the wearer, a second end portion 7 intended to be directed backwards on the wearer, a longitudinal centre line 8 extending in the longitudinal direction of the panty liner, and a transverse centre line 9 extending in the transverse direction of the panty liner. Centre line means a line which runs in the longitudinal or the transverse direction and is located at an equal distance from the long sides or, respectively, the short sides of the panty liner. The panty liner has an upper side 10 which is intended to face the wearer during use and a lower side 11 intended to face away from the wearer.

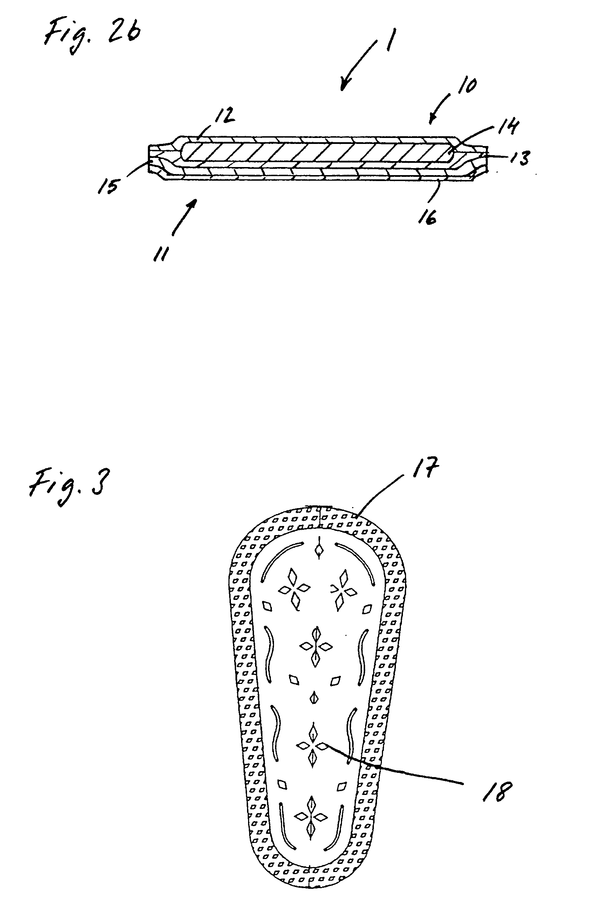

The panty liner comprises a liquid-permeable surface layer 12 arranged on the upper side so that it faces the wearer during use and a liquid-blocking backing layer 13 on the lower side. An absorption body 14 is arranged between the surface layer 12 and the backing layer 13. The surface layer and the backing layer are suitably connected to the absorption body by glue. The surface layer 12 and the backing layer 13 can have the same extent in the longitudinal and transverse directions as the absorption body 14, see Figure 2a. It is also possible to design the panty liner so that the surface layer 12 and the backing layer 13 have a greater extent than the absorption body 14. The surface layer and the backing layer are then interconnected in the region outside the absorption body around the periphery of the absorption body, see Fig. 2b, a "fin" being formed around the edge of the panty liner. In the manufacture of the panty liner, the various layers can during gluing be pressed together by an embossed roller, for example, around the edge 17 of the panty liner. It is also possible, during manufacture, to emboss a pattern 18 into the product, see Figure 3. Within the edge 17, the product is essentially of uniform thickness, which means that it has a plane shape in order that good contact against the outer parts of the genital area of the wearer is obtained.

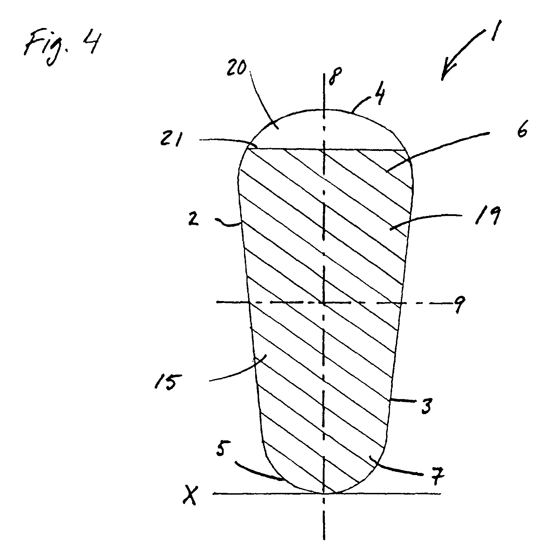

Figures 2a and 2b show sections through the panty liner in Figure 1 along the line II-II. Arranged on the lower side of the panty liner is a means of attachment in the form of pressure-sensitive adhesive 15. The adhesive 15 can cover the entire lower side, be applied in parallel strands along the lower side or be applied to the lower side in another suitable pattern such as, for example, a bar pattern or spots. A detachable protective layer 16 is arranged over the adhesive 15, and the protective layer 16 is removed by the wearer before the panty liner is fitted in the underwear of the wearer. The protective layer 16 can be, for example, what is known as a release paper which can consist of a paper layer coated with silicone. According to a preferred embodiment, the pressure-sensitive adhesive 15 is arranged in a first region 19 which covers the entire lower side with the exception of a second region 20, without adhesive, on the first end portion 6 furthest forward on the panty liner, see Figure 4. A detachable protective layer 16 is arranged over the entire lower side of the panty liner so that it covers both the region 19 with adhesive and the region 20 without adhesive. In this way, it is easy to remove the protective layer before fitting the panty liner, because that part of the protective layer which lies over the region without adhesive serves as a gripping flap which is easy to take hold of for removal. The extent of the region 20 without adhesive is suitably designed so that it extends from the edge furthest forward on the front short side to a line 21 which is parallel to the transverse centre line 9. The distance between the front edge and the line 21 suitably lies within the range 2-30 mm and the distance is preferably 5 mm. It is also possible to locate the region without adhesive in other parts of the panty liner, for example it is possible to arrange the region without adhesive on the other end portion 7 in the same manner as on the first end portion 6 or on both the first and the second end portion.

It should also be mentioned that it is possible, within the scope of the invention, to use other means of attachment, such as hook-and-loop or friction fasteners.

The surface material 12 can consist of any conventional material, for example a non-woven, a perforated plastic film or a laminate consisting of a perforated plastic film and a non-woven.

The backing layer 13 consists of a liquid-impermeable material such as, for example, a thin, liquid-impermeable plastic film, or of a material which in itself is liquid-permeable and is then coated with a layer of plastic, resin or another liquid-impermeable material. The barrier layer 13 can therefore consist of any material which meets the criterion of liquid-impermeability so that leakage from the lower side is prevented, and is sufficiently flexible and skin-friendly for the purpose. Examples of suitable materials are plastic films made of polyethylene, polyester or polypropylene, non-wovens and laminates consisting of non-wovens and plastic films. A backing layer made of a laminate consisting of a liquid-impermeable plastic layer facing the absorption body, and a non-woven facing the underwear of the wearer provides a leakproof barrier layer with a textile feel. It is also possible to design the backing of the panty liner with a breathable backing layer made of, for example, SMS (spunbond-meltblown-spunbond) or of a breathable plastic film made of, for example, polyethylene.

The absorption body 14 is suitably made from natural fibres such as, for example, cellulose pulp, absorbent synthetic fibres or mixtures of natural fibres and synthetic fibres. The absorption body 14 preferably consists of what is known as an airlaid cellulose body. It is also possible to mix what are known as superabsorbents into the absorption body. A superabsorbent is a polymer with the capacity to absorb several times its own weight of liquid. The absorption body 14 can also contain additional components such as shape-stabilizing means, liquid-spreading means or binders. It is also possible to use various types of absorbent foamed material in the absorption body.

An admission layer can be arranged between the surface layer and the absorption body. The purpose of the admission layer is to draw liquid into the absorbent product and to transport the liquid down to the absorption body 14. The admission layer can be made of, for example, a non-woven material of low density.

Trials have shown that the length of the panty liner is to lie within the range 65-100 mm and its maximum width in the transverse direction is to lie within the range 35-55 mm in order that its extent will not be too great but will still be sufficient to ensure good absorption. By virtue of the fact that the panty liner does not cover as great a part of the genital area as a conventional product of this type, good air permeation is obtained. In order for the panty liner to fit most types of briefs, even what are known as thongs, it should have a shape which tapers backwards, that is to say that the width in the transverse direction, parallel to the transverse centre line 9, of the second end portion 7 is smaller than the width in the transverse direction of the first end portion 6. In this respect, trials have shown that a suitable geometry of the panty liner is obtained when the width of the product in the transverse direction at a point, A, along the longitudinal centre line 8 which lies at a distance of 15% of the length of the product from an end line, X, see Figure 1, which runs in the transverse direction of the product, that is to say parallel to the transverse centre line 9, and is tangent to the second end portion 7 is 60-75% of the width of the product in the transverse direction at a point, B, along the longitudinal centre line 8 which lies at a distance of 80% of the length of the product from the end line, X. The fact that the width of the liner is defined in relation to points, A and B, on the longitudinal centre line 8 which lie at a distance of 15% and, respectively, 80% of the length of the product from the end line, X, is due to the fact that the panty liner can have end portions 6, 7 which are rounded. It is also possible to design the end portions 6, 7 with completely straight edges. The product preferably has rounded end portions in those parts of the end portions 6, 7 which, in the longitudinal direction, extend from the points A and B respectively and out towards the short sides 4, 5 of the panty liner. In such an embodiment, the panty liner is at its widest in the transverse direction at point B.

The length of the panty liner (or of the absorbent product) preferably lies within the range 84-98 mm or 89-93 mm, and its maximum width in the transverse direction preferably lies within the range 36-48 mm or 40-44 mm. According to an especially preferred embodiment, the product has a length of 91 mm and a maximum width in the transverse direction which is 42 mm. The width of the product in the transverse direction at the point, A, along the longitudinal centre line 8 which lies at a distance of 15% of the length of the product from the end line, X, is preferably 64-72% or 66-70% of the width of the product in the transverse direction at the point, B, along the longitudinal centre line 8 which lies at a distance of 80% of the length of the product from the end line, X. According to the especially preferred embodiment, the width of the product in the transverse direction at the point, A, along the longitudinal centre line 8 which lies at a distance of 15% of the length of the product from the end line, X, is 68% of the width of the product in the transverse direction at the point, B, along the longitudinal centre line 8 which lies at a distance of 80% of the length of the product from the end line, X.

The two long sides 2, 3 extend between the first end portion 6 and the second end portion 7. The two long sides are straight and somewhat inclined in relation to the longitudinal centre line 8, because the width in the transverse direction of the second end portion 7 is smaller than the width in the transverse direction of the first end portion 6. In order that the correct geometry of the panty liner is obtained, the inclination of the two long sides should be such that, for each 5 mm along the longitudinal centre line 8, from the second end portion 7 towards the first end portion 6, the width of the panty liner in the transverse direction will increase by 0.6-2.6 mm. According to the especially preferred embodiment, the increase is 1.1 mm for each 5 mm along the longitudinal centre line 8.

The invention is not to be considered as being limited to the embodiments above because these are intended only to explain the invention. It is also possible, within the scope of the invention, to combine features from different embodiments with one another.

## Claims

1. Absorbent product, such as a sanitary towel, an incontinence pad, a panty liner or the like, which product has a generally elongate shape with a longitudinal direction and a transverse direction and a longitudinal centre line (8) extending in the longitudinal direction, and has two long sides (2,3) two short sides (4,5), an upper side (10) and a lower side (11) and has a first end portion (6) intended to be directed forwards on the wearer and a second end portion (7) intended to be directed backwards on the wearer, the width in the transverse direction of the second end portion (7) being smaller than the width in the transverse direction of the first end portion (6), **characterized in that** the length of the product in the longitudinal direction is 65-100 mm, **in that** the maximum width of the product in the transverse direction is 35-55 mm, and **in that** the width of the product in the transverse direction at a point (A) along a longitudinal centre line (8) extending in the longitudinal direction which lies at a distance of 15% of the length of the product from an end line (X) which runs in the transverse direction of the product and is tangent to the second end portion (7) is 60-75% of the width of the product in the transverse direction at a point (B) along the longitudinal centre line (8) which lies at a distance of 80% of the length of the product from the end line (X), and **in that** the long sides (2,3) of the product are straight and inclined in relation to the longitudinal centre line (8) with an inclination that is such that, for each 5 mm along the longitudinal centre line (8), from the second end portion (7) towards the first end portion (6), the width of the panty liner in the transverse direction increases by 0.6-2.6 mm.

2. Absorbent product according to Claim 1, **characterized in that** the length of the product in the longitudinal direction is 84-98 mm.

3. Absorbent product according to Claim 1 or 2, **characterized in that** the length of the product in the longitudinal direction is 89-93 mm, preferably 91 mm.

4. Absorbent product according to any one of the preceding claims, **characterized in that** the maximum width of the product in the transverse direction is 36-48 mm.

5. Absorbent product according to any one of the preceding claims, **characterized in that** the maximum width of the product in the transverse direction is 40-44 mm, preferably 42 mm.

6. Absorbent product according to any one of the preceding claims, **characterized in that** the width of the product in the transverse direction at the point (A) along the longitudinal centre line (8) which lies at a distance of 15% of the length of the product from the end line (X) is 64-72% of the width of the product in the transverse direction at the point (B) along the longitudinal centre line (8) which lies at a distance of 80% of the length of the product from the end line (X).

7. Absorbent product according to any one of the preceding claims, **characterized in that** the width of the product in the transverse direction at the point (A) along the longitudinal centre line (8) which lies at a distance of 15% of the length of the product from the end line (X) is 66-70%, preferably 68%, of the width of the product in the transverse direction at the point (B) along the longitudinal centre line (8) which lies at a distance of 80% of the length of the product from the end line (X).

8. Absorbent product according to any one of the preceding claims, **characterized in that** the surface layer (12) and backing layer (13) of the product have the same extent in the longitudinal direction and the transverse direction as the absorption body (14) of the product.

9. Absorbent product according to any one of Claims 1-7, **characterized in that** the surface layer (12) of the product and the backing layer (13) of the product have an extent in the longitudinal direction and the transverse direction which is greater than that of the absorption body (14) of the product, the surface layer (12) and the backing layer (13) being interconnected in the region outside the absorption body (14).

10. Absorbent product according to any one of the preceding claims, **characterized in that** a first region (19), with adhesive (15), for securing the product in a pair of briefs, and a second region (20), without adhesive, are arranged on the lower side of the product, a detachable protective layer (16) being arranged over both the region (19) with adhesive and the region (20) without adhesive.

## Patentansprüche

1. Absorbierendes Produkt, wie beispielsweise eine Damenbinde, ein Inkontinenzpad, eine Slipeinlage oder ähnliches, welches Produkt eine im Wesentlichen längliche Form mit einer Längsrichtung und einer Querrichtung und eine Längsmittellinie (8), die sich in Längsrichtung erstreckt, aufweist und zwei lange Seiten (2, 3), zwei kurze Seiten (4, 5), eine Oberseite (10) und eine Unterseite (11) aufweist und einen ersten Endabschnitt (6), der dazu gedacht ist am Träger nach vorne zu weisen und einen zweiten Endabschnitt (7), der dazu gedacht ist am Träger nach hinten zu weisen, umfasst, wobei die Breite des zweiten Endabschnitt (7) in Querrichtung kleiner ist als die Breite des ersten Endabschnitts (6) in Querrichtung, **dadurch gekennzeichnet, dass** die Länge des Produkts in Längsrichtung 65-100 mm beträgt, **dadurch**, **dass** die maximale Breite des Produkts in Querrichtung 35-55 mm beträgt und **dadurch**, **dass** die Breite des Produkts in Querrichtung an einem Punkt (A) auf einer Längsmittellinie (8), die sich in Längsrichtung erstreckt und der in einem Abstand von 15% der Länge des Produkts von einer Endlinie (X), die in Querrichtung des Produkts verläuft und den zweiten Endabschnitt (7) berührt, 60-75% der Breite des Produkts in Querrichtung an einem Punkt (B) auf die Längsmittellinie (8), der in einem Abstand von 80% der Länge des Produkts von der Endlinie (X) liegt, entspricht und **dadurch, dass** die langen Seiten (2, 3) des Produkts gerade und in Bezug auf die Längsmittellinie (8) mit einer Neigung geneigt sind, die derart ist, dass die Breite der Slipeinlage in Querrichtung alle 5 mm entlang der Längsmittellinie (8) von dem zweiten Endabschnitt (7) in Richtung des ersten Endabschnitts (6) um 0,6-2,6 mm zunimmt.

2. Absorbierendes Produkt nach Anspruch 1, **dadurch gekennzeichnet, dass** die Länge des Produkts in Längsrichtung 84-98 mm beträgt.

3. Absorbierendes Produkt nach Anspruch 1 oder 2, **dadurch gekennzeichnet, dass** die Länge des Produkts in Längsrichtung 89-93 mm, vorzugsweise 91 mm beträgt.

4. Absorbierendes Produkt nach einem der vorstehenden Ansprüche, **dadurch gekennzeichnet, dass** die maximale Breite des Produkts in Querrichtung 36-48 mm beträgt.

5. Absorbierendes Produkt nach einem der vorstehenden Ansprüche, **dadurch gekennzeichnet, dass** die maximale Breite des Produkts in Querrichtung 40-44 mm, vorzugsweise 42 mm beträgt.

6. Absorbierendes Produkt nach einem der vorstehenden Ansprüche, **dadurch gekennzeichnet, dass** die Breite des Produkts in Querrichtung an dem Punkt (A) auf der Längsmittellinie (8), der in einem Abstand von 15% der Länge des Produkts von der Endlinie (X) liegt, 64-72% der Breite des Produkts in Querrichtung am Punkt (B) auf der Längsmittellinie (8), der in einem Abstand von 80% der Länge des Produkts von der Endlinie (X) liegt, beträgt.

7. Absorbierendes Produkt nach einem der vorstehenden Ansprüche, **dadurch gekennzeichnet, dass** die Breite des Produkts in Querrichtung an dem Punkt (A) auf der Längsmittellinie (8), der in einem Abstand von 15% der Länge des Produkts von der Endlinie (X) liegt 66-70%, vorzugsweise 68% der Breite des Produkts in Querrichtung am Punkt (B) auf der Längsmittellinie (8), der in einem Abstand von 80% der Länge des Produkts von der Endlinie (X) liegt, beträgt.

8. Absorbierendes Produkt nach einem der vorstehenden Ansprüche, **dadurch gekennzeichnet, dass** die Oberflächenlage (12) und die Decklage (13) des Produkts die gleiche Dimension in Längsrichtung und Querrichtung wie der Absorptionskörper (14) des Produkts aufweisen.

9. Absorbierendes Produkt nach einem der Ansprüche 1-7, **dadurch gekennzeichnet, dass** die Oberflächenlage (12) des Produkts und die Decklage (13) des Produkts eine Dimension in Längsrichtung und Querrichtung aufweisen, die größer ist als die des Absorptionskörpers (14) des Produkts, wobei die Oberflächenlage (12) und die Decklage (13) im Bereich außerhalb des Absorptionskörpers (14) verbunden sind.

10. Absorbierendes Produkt nach einem der vorstehenden Ansprüche, **dadurch gekennzeichnet, dass** ein erster Bereich (19) mit Klebemittel (15) zum Befestigen des Produkts in einem Höschen und ein zweiter Bereich (20) ohne Klebemittel auf der Unterseite des Produkts angeordnet sind und eine lösbare Schutzschicht (16) über sowohl dem Bereich (19) mit dem Klebemittel als auch dem Bereich (20) ohne das Klebemittel angeordnet ist.

## Revendications

1. Produit absorbant, tel qu'une serviette hygiénique, une serviette d'incontinence, un protège-slip ou similaire, lequel produit a une forme généralement allongée avec une direction longitudinale et une direction transversale, et une ligne centrale longitudinale (8) s'étendant dans la direction longitudinale, et a deux côtés longs (2, 3), deux côtés courts (4, 5), un côté supérieur (10) et un côté inférieur (11), et a une première partie d'extrémité (6) destinée à être dirigée vers l'avant sur l'utilisateur et une deuxième partie d'extrémité (7) destinée à être dirigée vers l'arrière sur l'utilisateur, la largeur dans la direction transversale de la deuxième partie d'extrémité (7) étant inférieure à la largeur dans la direction transversale de la première partie d'extrémité (6), **caractérisé en ce que** la longueur du produit dans la direction longitudinale est de 65 à 100 mm, **en ce que** la largeur maximale du produit dans la direction transversale est de 35 à 55 mm, et **en ce que** la largeur du produit dans la direction transversale en un point (A) le long d'une ligne centrale longitudinale (8) s'étendant dans la direction longitudinale qui repose à une distance de 15 % de la longueur du produit à partir d'une ligne d'extrémité (X) qui se dirige dans la direction transversale du produit et est tangente à la deuxième partie d'extrémité (7) est de 60 à 75 % de la largeur du produit dans la direction transversale en un point (B) le long de la ligne centrale longitudinale (8) qui repose à une distance de 80 % de la longueur du produit à partir de la ligne d'extrémité (X), et **en ce que** les côtés longs (2, 3) du produit sont droits et inclinés par rapport à la ligne centrale longitudinale (8), avec une inclinaison qui est telle que, tous les 5 mm le long de la ligne centrale longitudinale (8), de la deuxième partie d'extrémité (7) à la première partie d'extrémité (6), la largeur du protège-slip dans la direction transversale augmente de 0,6 à 2,6 mm.

2. Produit absorbant selon la revendication 1, **caractérisé en ce que** la longueur du produit dans la direction longitudinale est de 84 à 98 mm.

3. Produit absorbant selon la revendication 1 ou 2, **caractérisé en ce que** la longueur du produit dans la direction longitudinale est de 89 à 93 mm, de préférence de 91 mm.

4. Produit absorbant selon l'une quelconque des revendications précédentes, **caractérisé en ce que** la largeur maximale du produit dans la direction transversale est de 36 à 48 mm.

5. Produit absorbant selon l'une quelconque des revendications précédentes, **caractérisé en ce que** la largeur maximale du produit dans la direction transversale est de 40 à 44 mm, de préférence de 42 mm.

6. Produit absorbant selon l'une quelconque des revendications précédentes, **caractérisé en ce que** la largeur du produit dans la direction transversale au point (A) le long de la ligne centrale longitudinale (8) qui repose à une distance de 15 % de la longueur du produit à partir de la ligne d'extrémité (X) est de 64 à 72 % de la largeur du produit dans la direction transversale au point (B) le long de la ligne centrale longitudinale (8) qui repose à une distance de 80 % de la longueur du produit à partir de la ligne d'extrémité (X).

7. Produit absorbant selon l'une quelconque des revendications précédentes, **caractérisé en ce que** la largeur du produit dans la direction transversale au point (A) le long de la ligne centrale longitudinale (8) qui repose à une distance de 15 % de la longueur du produit à partir de la ligne d'extrémité (X) est de 66 à 70 %, de préférence de 68 %, de la largeur du produit dans la direction transversale au point (B) le long de la ligne centrale longitudinale (8), qui repose à une distance de 80 % de la longueur du produit à partir de la ligne d'extrémité (X).

8. Produit absorbant selon l'une quelconque des revendications précédentes, **caractérisé en ce que** la couche superficielle (12) et la couche de renfort (13) du produit ont la même étendue dans la direction longitudinale et dans la direction transversale que le corps d'absorption (14) du produit.

9. Produit absorbant selon l'une quelconque des revendications 1 à 7, **caractérisé en ce que** la couche superficielle (12) du produit et la couche de renfort (13) du produit ont une étendue dans la direction longitudinale et la direction transversale qui est supérieure à celle du corps d'absorption (14) du produit, la couche superficielle (12) et la couche de renfort (13) étant interconnectées dans la région à l'extérieur du corps d'absorption (14).

10. Produit absorbant selon l'une quelconque des revendications précédentes, **caractérisé en ce qu'**une première région (19), avec un adhésif (15), pour fixer le produit dans une paire de caleçons, et une deuxième région (20), sans adhésif, sont agencées sur le côté inférieur du produit, une couche protectrice détachable (16) étant agencée au-dessus de la région (19) avec adhésif et de la région (20) sans adhésif.
